# EUROPÄISCHE PATENTANMELDUNG

(11) **EP 0 555 642 A2**
(43) Veröffentlichungstag der Anmeldung: **18.08.1993**
(21) Anmeldenummer: 93100384.2
(22) Anmeldetag: 13.01.1993
(51) Int. Cl.: A61M 31/00

(54) **Vorrichtung zur Füllung der Hohlorgane von Lebewesen mit physiologisch verträglichem gasförmigem Medium**

(30) Priorität: 14.01.1992 DE 9200330 U; 30.04.1992 DE 9205798 U
(71) Anmelder: Schmitz-Rode, Thomas, D-52070 Aachen (DE); Alzen, Gerhard, Dr., D-52074 Aachen (DE)
(72) Erfinder: Schmitz-Rode, Thomas, D-52070 Aachen (DE); Alzen, Gerhard, Dr., D-52074 Aachen (DE)
(74) Vertreter: Selting, Günther, Dipl.-Ing.

(57) **Zusammenfassung**

Eine Vorrichtung zur Füllung der Hohlorgane von Lebewesen mit physiologisch verträglichem gasförmigem Medium zu diagnostischen oder therapeutischen Zwecken weist einen mit hochreinem sterilem und unter Druck befindlichem Gas gefüllten, handlich kleinen Druckbehälter (10) mit einem absperrbaren Auslaß (15) auf. Die Vorrichtung weist ferner eine Gasvolumen-Reguliervorrichtung (20) mit einer Einlaß- und einer Auslaßseite auf, wobei die Einlaßseite an den Auslaß (15) des Druckbehälters (10) angeschlossen ist. Eine Applikatorleitung (27) ist mit der Auslaßseite der Gasvolumen-Reguliervorrichtung (20) verbindbar.

## Beschreibung

Die Erfindung bezieht sich auf eine Vorrichtung zur Füllung der Hohlorgane von Lebewesen mit physiologisch verträglichem gasförmigem Medium, wie Kohlendioxid (Co₂); Helium (HE) oder Stickoxid (N₂O) zu diagnostischen oder therapeutischen Zwecken.

Kohlendioxid (Co₂) eignet sich als alternatives Kontrastmittel zur intraarteriellen und eingeschränkt intravenösen Gefäßdarstellung bei Patienten, die jodhaltiges flüssiges Kontrastmittel nicht tolerieren. Bisher wird von dieser Möglichkeit bei der Angiographie Gebrauch gemacht. Dabei wird Kohlendioxid aus einer großen stehenden Standard-Druckflasche in eine Spritze abgefüllt, die einen verschließbaren Spritzenkonus hat, so daß das in den Hubraum der Spritze eingezogene drucklose (in Umgebungsdruck befindliche) Kohlendioxid bis zur Applikation in der Spritze verbleibt. An die Verschlußvorrichtung des Spritzenkonus setzt der Anwender einen Katheter an und injiziert manuell das drucklose Kohlendioxid in das Gefäßsystem eines Patienten. Die manuelle Injektion hat bei der Angiographie den Nachteil, daß man keine Kontrolle darüber hat, ob eventuell teilweise Luft injiziert wird und daß die manuelle Injektion nicht unbedingt in dem für eine gute Gefäßdarstellung erforderlichen optimalen Bereich der Parameter Volumenstrom, Volumen und Druck erfolgt. Da das Kohlendioxid in der Spritze drucklos ist, hängen die erwähnten Parameter von der Wahl der Spritzengröße, des Katheterlumens und vor allem von dem Kraftaufwand und der Gleichförmigkeit ab, mit der der Anwender den Spritzenkolben betätigt, so daß nicht in definierter Zeiteinheit eine definierte Menge appliziert wird und die Applikationen nicht reproduzierbar sind. Bei ungleichförmigem Kolbenvorschub reißt der Kohlendioxid-Bolus ab und es ergibt sich eine diskontinuierliche Gefäßdarstellung, die das Untersuchungsergebnis beeinträchtigt. Außerdem erfordert das Totvolumen der Spritze eine umständliche, gegebenenfalls mehrfache, Kohlendioxidspülung, die von einer Hilfskraft des Anwenders durchgeführt werden muß. Als weitere Hilfsarbeit kommt hinzu, daß die Standard-Druckflasche im Auslaßbereich als auch die Überleitungsorgane zur Spritze hin vor jedem Anwendungsfall für eine Füllung unter sterilen Bedingungen präpariert werden muß.

Die niedrige Viskosität des Gases im Vergleich zu flüssigen Kontrastmitteln erlaubt auch eine vorteilhafte Anwendung als Repositionsmedium zur Lösung von Darminvaginationen und als negatives Kontrastmittel zur Doppelkontrastdarstellung des Dünndarmes. Die schnelle Resorption von Kohlendioxid aus dem Darmlumen ermöglicht ebenso eine vorteilhafte Anwendung bei der Doppelkontrastdarstellung des Colons, da hierdurch die durch die Darmblähung bedingten Beschwerden des Patienten nach der Untersuchung gemildert werden. Für diese Zwecke gibt es bisher keine geeignete Applikationsvorrichtung.

Der Erfindung liegt die Aufgabe zugrunde, eine Vorrichtung zur Füllung beliebiger Hohlorgane von Mensch und Tier mit gasförmigem Kohlendioxid oder anderen körperverträglichen Gasen zu schaffen, die bei einfacher Handhabung reproduzierbare applizierte Gasvolumina ermöglicht.

Diese Aufgabe wird erfindungsgemäß gelöst durch einen mit hochreinem sterilem und unter Druck befindlichem Gas gefüllten, handlich kleinen Druckbehälter, der einen absperrbaren Auslaß aufweist, eine Gasvolumen-Reguliervorrichtung mit einer Einlaß- und einer Auslaßseite, wobei die Einlaßseite an den Auslaß des Druckbehälters angeschlossen ist, und eine mit der Auslaßseite der Gasvolumen-Reguliervorrichtung verbindbare Applikatorleitung.

Eine solche Vorrichtung bildet eine einfach handhabbare Einheit, die als preiswertes Einmalprodukt herstellbar ist und sich in steriler Verpackung lagern und versenden läßt, was insbesondere für die angiographische Anwendung bedeutungsvoll ist. Das unter Druck aus der Applikatorleitung ausströmende Gasvolumen kann entweder dadurch begrenzt werden, daß die mit dem Auslaß des Druckbehälters verbundene Gasvolumen-Reguliervorrichtung über einen vorgegebenen Zeitabschnitt einen Durchgang zur Applikatorleitung öffnet, so daß für weitere Injektionen Gas im Druckbehälter verbleibt oder es kann der Behälter so klein dimensioniert werden, daß er bereits bei einem Injektionsvorgang entleert wird oder es kann eine mit einer Dosiervorrichtung erreichbare zeitunabhängige Volumenbemessung vorgesehen werden. Mit Hilfe einer Druckmeßvorrichtung kann der Druck des applizierten Gases kontrolliert bzw. eingestellt werden. Druck und Volumenstrom im Bereich der Austrittsöffnung der Applikatorleitung sind ebenfalls durch die Gasvolumen-Reguliervorrichtung so einstellbar, daß sie den physiologischen Erfordernissen für eine effiziente, aber sichere Darstellung entsprechen. Da das Gas mit meßbarem und einstellbarem Eigendruck appliziert wird, sind die Parameter der Gasapplikation reproduzierbar. Es werden in definierter Zeit definierte Mengen abgegeben, so daß der Bolus nicht zerreißt und eine für alle Anwendungszwecke günstige kontinuierliche Darstellung erreicht wird.

Die Gasvolumen-Reguliervorrichtung ist für die Anwendung der Füllung des Darmes oder krankhafter Hohlraumbildungen aus einem Drosselstück gebildet, mit dem ein in eine Leitung zwischen Druckbehälter und Drosselstück eingesetztes Steuerventil zusammenwirkt. Das Steuerventil kann bei einer einfachen Ausführung der Vorrichtung von Hand betätigbar sein. In diesem Fall wird das aus der Applikatorleitung unter Druck ausströmende Gasvolumen durch die Länge der Betätigung des Steuerventils, z.B. Knopfdruck, bestimmt. Bei Verwendung eines relativ großen Druckbehälters ergibt sich allerdings keine absolut sichere Begrenzung des applizierten Gasvolumens, da die Zeitspanne der Steuerventilbetätigung in Abhängigkeit vom Untersuchenden sehr unterschiedlich ausfallen kann. Aufgrund der im Vergleich zu flüssigen Kontrastmitteln niedrigen Viskosität von Gasen kann es bei Gabe eines zu hohen Volumens des Gases zu einer unkontrollierten Ausbreitung kommen. Wird das applizierte Volumen durch Verwendung eines angepaßt kleinen Druckbehälters begrenzt, der sich bereits bei einem Injektionsvorgang entleert, so besteht der Nachteil darin, daß für jeden wiederholten Injektionsvorgang ein neuer Druckbehälter verwendet werden muß.

Mehrfachinjektionen in kontinuierlicher Folge sind somit nicht möglich.

Eine weitere Reguliermöglichkeit ergibt sich dadurch, daß anstatt eines Drosselstückes mit unveränderlichem Durchgangsquerschnitt ein Drosselstück mit veränderlichem Druchgangsquerschnitt verwendet wird. Als Drosselstück mit unveränderlichem Durchgangsquerschnitt kann jede Leitung gelten, deren Durchmesser kleiner ist als der Querschnitt des Auslasses des Druckbehälters, so daß der Druck des das Drosselstück durchströmenden Gases abnimmt und einen körperverträglichen Wert erhält, der sich während der Gesamtzeit der Applikation in tolerablen Grenzen bewegt.

Insbesondere zur Darstellung des Gefäßsystems empfiehlt sich eine sichere Begrenzung des applizierten Volumens und die Ermöglichung von Mehrfachinjektionen. Zu diesem Zweck ist die Gasvolumen-Reguliervorrichtung aus einem Dreiwegehahn mit T-förmigen Anschlüssen und einem Küken mit L-förmigem Durchgang sowie einer mit einem der Anschlüsse verbundenen Gas-Dosiervorrichtung gebildet. Es können mit einem leichten, portablen und einfach handhabbaren Kohlendioxid-Injektor Gefäßdarstellungen vorgenommen werden, wobei eine zwischengeschaltete Gas-Dosiervorrichtung eine sichere Begrenzung des applizierten Gasvolumens erlaubt, derart, daß der Inhalt des Druckbehälters in mehreren weitgehend identischen Gasportionen (Volumen, Druck) appliziert werden kann. Da die abzugebende Gasmenge sich nach dem Anwendungszweck richtet, ist vorteilhafterweise die Gas-Dosiervorrichtung mit einer Dosierkammer mit veränderlichem Volumen versehen.

Als Beispiel für eine zweckmäßige Ausgestaltung der Erfindung wird die Dosiervorrichtung als zylindrischer Hohlkörper ausgebildet, der an einer Stirnseite einen Konnektor zur Verbindung mit dem einen Anschluß des Dreiwegehahns der Gasvolumen-Reguliervorrichtung aufweist und in dem ein Kolben verstellbar ist, der in verschiedenen Positionen arretierbar ist. Das vorzugsweise lediglich in zwei Stellungen drehbare Küken des Dreiwegehahns schließt eine irrtümliche Fehlbedienung aus. In einer Stellung des Kükens gelangt Gas aus den Druckbehälter in die Dosierkammer und in der zweiten Stellung des Kükens entleert sich das Gasvolumen der Dosierkammer selbsttätig unter Druck in das Gefäßsystem, wodurch eine gleichmäßige, zusammenhängende und reproduzierbare Gefäßdarstellung erzeugt wird. Die gesamte Einheit ist einfach sterilisierbar. Eine Gasdruck-Meßvorrichtung an der Gas-Dosiervorrichtung erlaubt die Überwachung und Regulierung des Druckes des zu applizierenden Gases.

Die Veränderung der Größe der Dosierkammer durch Verschiebung des Kolbens in dem zylindrischen Hohlkörper macht es möglich, durch vollständiges Einfahren des Kolbens zu Beginn der ersten Füllung der Dosierkammer ein nicht gasgefülltes Totvolumen zu vermeiden. Sodann kann der Kolben so eingestellt und arretiert werden, daß das Volumen den jeweiligen angiographischen Erfordernissen, z.B. der Übersichtsaortographie, der selektiven Gefäßdarstellung und ähnlichem angepaßt ist.

Je nach Anwendungszweck dient als Applikatorleitung eine Darmsonde, ein Katheter oder eine Hohlnadel (bzw. Kanüle), die mittels eines Konnektors mit der Auslaßseite der Gasvolumen-Reguliervorrichtung verbindbar sind.

In der Zeichnung sind Ausführungsbeispiele der Erfindung schematisch dargestellt. Es zeigt:
- Fig. 1: ein Schema einer Gas-Applikationsvorrichtung mit einer Ausführungsform der Gasvolumen-Reguliervorrichtung,
- Fig. 2: eine apparative Umsetzung des Schemas nach Fig. 1,
- Fig. 3: ein Schema einer Gas-Applikationsvorrichtung mit einer zweiten Ausführungsform der Gasvolumen-Reguliervorrichtung,
- Fig. 4: ein Ausschnitt des Schemas nach Fig. 3 mit veränderter Dreiwegehahnstellung,
- Fig. 5: eine apparative Umsetzung des Schemas nach Fign. 3 und 4 und
- Fig. 6: die Apparatur nach Fig. 5 in steril verpacktem Zustand.

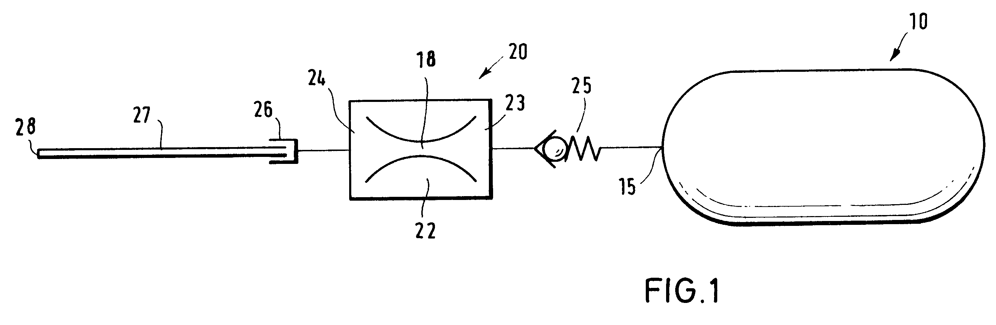

Das in Fig. 1 gezeigte Schema veranschaulicht einen Druckbehälter 10 handlicher Größe, der aus Metall oder Kunststoff bestehen kann und dessen Wandung Drücke von bis zu 20 bar aushalten muß. Der Druckbehälter 10, der als Flasche gestaltet sein kann, weist einen absperrbaren Auslaß 15 auf, an den eine Gasvolumen-Reguliervorrichtung 20 angeschlossen ist. Gemäß dem Schema besteht die Gasvolumen-Reguliervorrichtung 20 aus einem eine Drosselstelle 18 aufweisenden Drosselstück 22 mit einem Eingang 23 und einem Ausgang 24 und aus einem Steuerventil 25, das zwischen den Auslaß 15 des Druckbehälters 10 und den Eingang 23 des Drosselstückes 22 eingesetzt ist. Das Steuerventil 25 kann elektronisch betätigbar sein, damit exakte Öffnungszeiten erreichbar sind. Für die preiswerte Ausbildung der Vorrichtung als Wegwerfartikel wird ein handbetätigbares Steuerventil verwendet.

An den Ausgang 24 des Drosselstückes 22 ist über einen Konnektor 26 ein Katheter oder eine Darmsonde 27 angeschlossen, aus deren Austrittsöffnung 28 das Gas austritt.

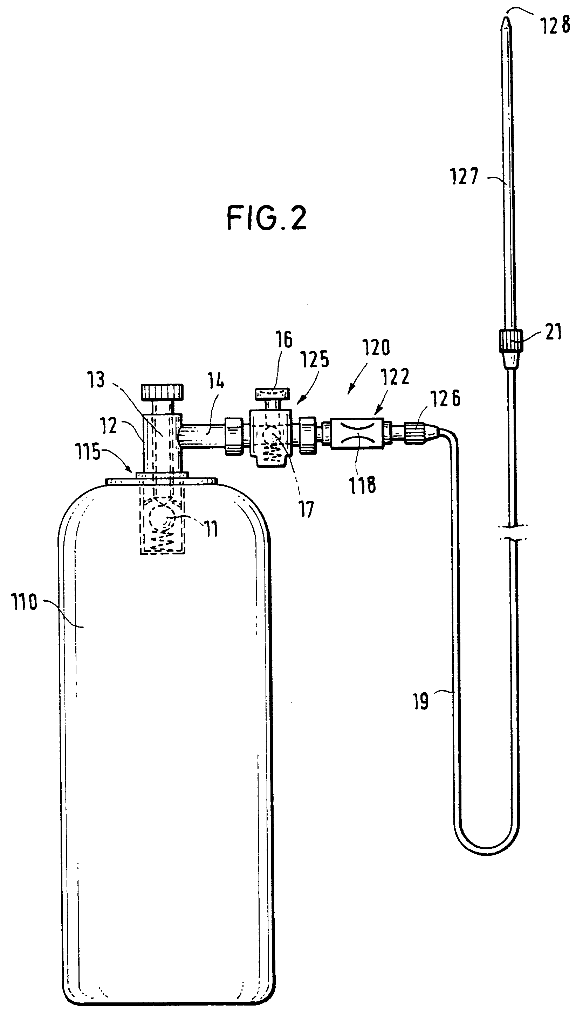

Bei dem in Fig. 2 gezeigten praktischen Ausführungsbeispiel des Schemas nach Fig. 1 ist der mit hochreinem, sterilem und unter Druck befindlichem Gas, z.B. Kohlendioxid, Helium oder Stickoxid, gefüllte Druckbehälter 110 eine handliche Metalldose, die z.B. ein Füllvolumen von 400 ml haben kann. In den Auslaß 115 des Druckbehälters 110 ist ein Rückschlagventil 11 eingebaut, das den Druckbehälter 110 nach außen abschließt. Mit Hilfe einer auf den Auslaß 115 aufgesetzten Betätigungsvorrichtung 12, in der ein axial verstellbarer, z.B. schraubbarer Zapfen 13 angeordnet ist, läßt sich das Rückschlagventil 11 öffnen. Von der Betätigungsvorrichtung 12 geht ein seitlicher Stutzen 14 aus, an dem das Steuerventil 125 (Öffner/Schließer) befestigt ist, das ein mit Hilfe eines Druckknopfmechanismus 16 betätigbares Rückschlagventil 17 aufweist. Mit dem Steuerventil 125 ist das Drosselstück 122 gekoppelt, welches mit dem Steuerventil 125 gemeinsam die Gasvolumen-Reguliervorrichtung 120 bildet. Das Drosselstück 122 enthält die Drosselstelle 118, deren Durchgang enger ist als der Querschnitt des Leitungssystems zwischen dem Druckbehälter 110 und dem Drosselstück 122. An der Auslaßseite des Drosselstückes 122 befindet sich ein Konnektor 126 mit einem Schlauch 19, mit dessen freiem Ende über einen weiteren Konnektor 21 die Sonde 127 gekoppelt ist.

Der Druckbehälter 110 enthält unter Druck befindliches, hochreines steriles körperverträgliches Gas, z.B. Kohlendioxid, in mindestens der für die jeweilige Untersuchung erforderlichen Gesamtmenge. Durch Hineinschrauben des Zapfens 13 wird das Rückschlagventil 11 des Druckbehälters 110 geöffnet, so daß das Gas in dem Stutzen 14 gegen das Steuerventil 125 ansteht. Bei Herunterdrücken des Druckknopfmechanismus 16 öffnet das Rückschlagventil 17 die Leitung, und das Gas durchströmt das Steuerventil 125, die Drosselstelle 118, den Konnektor 126, den Schlauch 19 und die Darmsonde 127. Die Drosselstelle 118 ist so ausgelegt, daß der passierende Volumenstrom die für eine optimale Untersuchung notwendige Größe hat. Der Druck in dem Druckbehälter 110 ergibt sich aus dem an der Austrittsöffnung 128 der Sonde 127 herrschenden optimalen Druck für die entsprechende Untersuchung zuzüglich der Druckverluste über der Darmsonde 127, dem Schlauch 19, dem Drosselstück 122 und dem Steuerventil 125. Die Größe des Druckbehälters 110 ergibt sich aus dem Behälterdruck und der insgesamt benötigten Gasmenge.

Für jeden Anwendungsfall wird eine speziell dimensionierte Vorrichtung bereitgestellt, wobei Volumen und Innendruck des Druckbehälters 110 sowie die Druckverlust-/Durchflußcharakteristik der Drosselstelle 118 des Drosselstückes 122 so aufeinander abgestimmt sind, daß die für die Untersuchung optimalen Parameter an der Austrittsöffnung 128 der Sonde 127 resultieren. Jede Vorrichtung ist zum einmaligen Gebrauch bestimmt. Dabei kann durch mehrmaliges Betätigen des Druckknopfes 16 des Steuerventils 125 der Inhalt des Druckbehälters 110 portionsweise abgegeben werden. Allerdings hängt die Dauer der Offenhaltung des Steuerventils 125 von dem Ermessen des Untersuchenden ab und ist nicht absolut gleichbleibend.

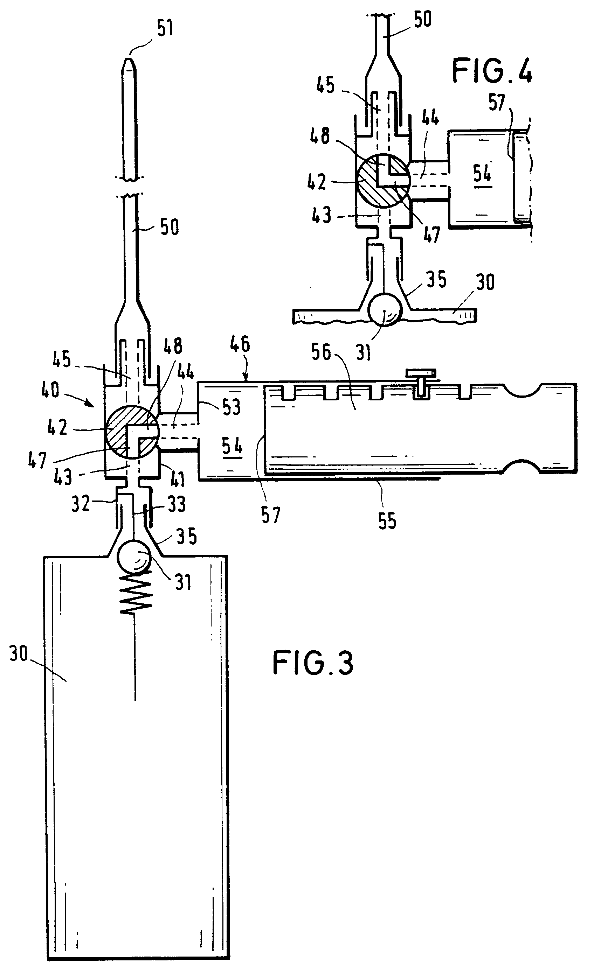

Das Schema gemäß Fign. 3 und 4 veranschaulicht einen Druckbehälter 30, der mit hochreinem sterilen und unter Druck befindlichem Gas, z.B. Kohlendioxid, Helium oder Stickoxid gefüllt ist und dessen Auslaß 35 ein Rückschlagventil 31 aufweist. An den Auslaß 35 ist eine Gasvolumen-Reguliervorrichtung 40 mit Hilfe eines Konnektors 32 angeschlossen, der einen axialen Dorn 33 trägt, welcher gegen den Ventilkörper des Rückschlagventiles 31 drückt, um den Auslaß 35 zu öffnen. Die Gasvolumen-Reguliervorrichtung 40 weist einen Dreiwegehahn 41 mit drei T-förmig angeordneten Anschlüssen 43,44,45 auf, von denen der Anschluß 44 mit einer Gas-Dosiervorrichtung 46 verbunden ist. In den Dreiwegehahn 41 ist ein Küken 42 mit L-förmigem Durchgang 47,48 in einem 90°-Bereich zwischen zwei Stellungen drehbar angeordnet. Mit dem Anschluß 45 ist ein Katheter 50 mit einer endständigen Auslaßöffnung 51 verbunden. Die Gas-Dosiervorrichtung 46 besteht aus einem zylindrischen Hohlkörper 55, in dem ein Kolben 56 verschiebbar ist, dessen Stirnfläche 57 mit der Endfläche 53 des zylindrischen Hohlkörpers 55 eine Dosierkammer 54 bildet. Die Größe der Dosierkammer 54 kann durch Verschieben und Arretieren des Kolbens 56 verändert werden.

Fig. 3 zeigt die erste Stellung des Kükens 42 des Dreiwegehahns 41 der Gasvolumen-Reguliervorrichtung 40. In diesem Fall sind die Durchgänge 47 und 48 des Kükens 42 mit den Anschlüssen 43 und 44 verbunden, die Gas aus dem Druckbehälter 30 in die Dosierkammer 54 leiten. Nach Füllung der Dosierkammer 54 wird das Küken 42 in die zweite Stellung nach Fig. 4 gedreht, in der die Durchgänge 47,48 mit den Anschlüssen 44 und 45 in Fluiddurchlaßverbindung stehen. Die aus der Dosierkammer 54 austretende Menge an Gas gelangt durch den Kanal 44,47,48, 45 in den Katheter 50 und wird von diesem zum Zielort geführt.

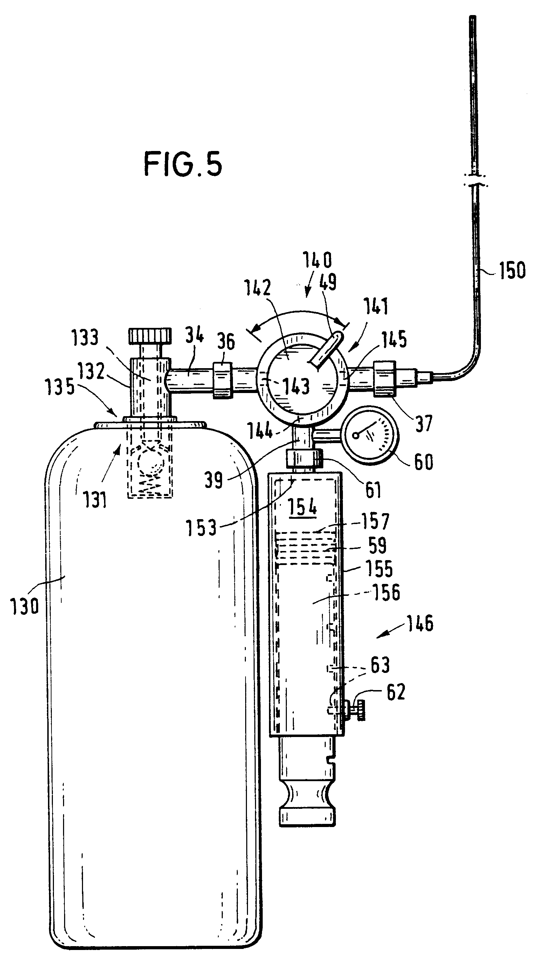

Das Schema nach Fign. 3 und 4 ist in dem Ausführungsbeispiel nach Fig. 5 realisiert. Der Druckbehälter 130 in Form einer handlichen Metalldose oder -flasche ist an seinem Auslaß 135 mit dem Rückschlagventil 131 ausgerüstet, das von einer Betätigungsvorrichtung gegen Federkraft öffenbar ist. Die Betätigungsvorrichtung besteht aus einem in eine Hülse 132 eingeschraubten Gewindezapfen 133, der in Grundstellung der Vorrichtung soweit aus der Hülse 132 herausgeschraubt ist, daß das Rückschlagventil 131 geschlossen ist. Mit der Hülse 132 ist ein seitlicher Stutzen 34 verbunden, an den mittels einer Schraubmutter 36 mit Dichtungen die Gasvolumen-Reguliervorrichtung 140 angeschlossen ist. Die Gasvolumen-Reguliervorrichtung 140 besteht aus dem Dreiwegehahn 141 gemäß dem Schema des Dreiwegehahns 40 in Fign. 3 und 4, dessen Küken 142 mit Hilfe eines Hebels 49 in die beiden Stellungen gemäß Fig. 3 oder Fig. 4 umschaltbar ist. Über einen Luer-Adapter ist mittels Verschraubungen der Diagnostik-Katheter 150 angeschlossen, der für die Gas-Angiographie bestimmt ist.

Während der Diagnostik-Katheter 150 mit dem Anschluß 145 des Dreiwegehahns 141 in Verbindung steht, ist der Anschluß 144 mit einem Übergangsstück 39 gekoppelt, an das eine Gasdruck-Meßvorrichtung 60 angesetzt ist. Das Übergangsstück 39 ist mittels einer Schraubverbindung 61 mit dem zylindrischen Hohlkörper 155 der Dosiervorrichtung 146 konnektiert, dessen eines Ende über eine Öffnung mit der Dosierkammer 154 in Verbindung steht. In dem Hohlkörper 155 ist der Kolben 156 verschiebbar, an dessen Stirnfläche 157 ein umfangsmäßiges Dichtungsteil 59 angrenzt. Zur Veränderung der Größe der Dosierkammer 154 kann der Kolben 156 in dem zylindrischen Hohlkörper 155 axial verschoben werden. Ein axial beweglicher Bolzen 62, der mit einer Längsreihe von Vertiefungen 63 in dem Kolben 156 beliebig zusammensteckbar ist, arretiert den Kolben 156 in der jeweils gewünschten Position.

Das Rückschlagventil 131, das als Kugelrückschlagventil gestaltet sein kann, schließt den Auslaß 135 nach Befüllung des Druckbehälters 130 mit hochreinem sterilem Gas selbsttätig. Zur Benutzung der Vorrichtung wird der Zapfen 133 in den Stutzen 132 hineingedreht und die Kugel des Rückschlagventils 131 wird behälterwärts gedrückt, so daß der Auslaß 135 freigegeben ist. Gleichzeitig wird eine gasdichte Verbindung zwischen dem Druckbehälter 130 und dem Dreiwegehahn 141 hergestellt. Das Küken 142 ist so eingestellt, daß das Gas in Richtung der Gas-Dosiervorrichtung 146 strömt. Vor der ersten Füllung der Dosierkammer 154 mit Gas ist der Kolben 156 vollständig gegen die Endfläche 153 des Hohlkörpers 155 vorgeschoben, so daß das Volumen der Dosierkammer 154 gleich null ist. Auf diese Weise wird ein initiales luftgefülltes Totvolumen vermieden. Sodann wird das gewünschte Volumen der Dosierkammer 154 mit Hilfe des Kolbens 156 eingestellt und die Dosierkammer füllt sich mit Gas, bis ein Druckgleichgewicht hergestellt ist. Dieser Zustand ist an der Gasdruck-Meßvorrichtung 60 ablesbar. Nach Umstellung des Hebels 49 des Dreiwegehahns 141 ist die Dosierkammer 154 mit dem Diagnostik-Katheter 150 verbunden. Gas strömt aus der Dosierkammer 154 durch den Diagnostik-Katheter 150 in das Gefäßsystem, bis ein Druckgleichgewicht zwischen physiologischem Druck im Gefäßsystem und dem Restdruck in der Dosierkammer 154 hergestellt ist. Wenn das Volumen des Druckbehälters 130 es zuläßt, kann der Vorgang des Einbringens einer bestimmten Menge an Gas in die Dosierkammer 154 und der Abgabe dieser dosierten Menge in das Gefäßsystem so oft wie nötig wiederholt werden.

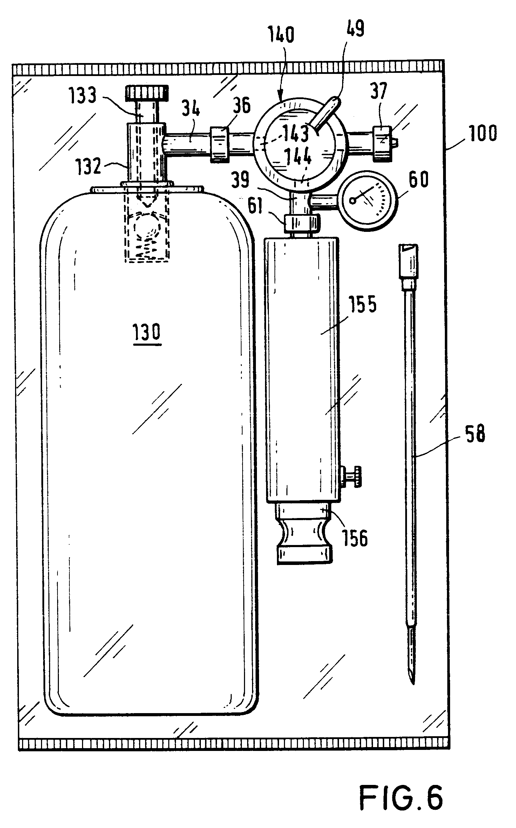

Fig. 6 zeigt die Vorrichtung nach Fig. 5 in steril verpacktem Zustand. Der Kolben 156 ist zur Vermeidung von Totvolumen bis zum Anschlag in den zylindrischen Hohlkörper 155 hineingedrückt und der Gewindezapfen 133 der Betätigungsvorrichtung des Rückschlagventils ist so weit wie zum Verschluß nötig aus der Hülse 132 herausgeschraubt. Anstatt eines Katheters, der in langer schmaler separater Verpackung geliefert wird, ist eine Hohlnadel-/Kanülenkombination 58 mit verpackt, die über einen Verbindungsschlauch an den Luer-Adapter 37 angeschlossen wird. Alle Teile sind gemeinsam in einer sterilen Folienverpackung 100 untergebracht.

## Patentansprüche

1. Vorrichtung zur Füllung der Hohlorgane von Lebewesen mit physiologisch verträglichem gasförmigem Medium zu diagnostischen oder therapeutischen Zwecken,
**gekennzeichnet durch**
- einen mit hochreinem sterilem und unter Druck befindlichem Gas gefüllten, handlich kleinen Druckbehälter (10;30), der einen absperrbaren Auslaß (15;35) aufweist,
- eine Gasvolumen-Reguliervorrichtung (20;40) mit einer Einlaß- und einer Auslaßseite, wobei die Einlaßseite an den Auslaß (15;35) des Druckbehälters (10;30) angeschlossen ist, und
- eine mit der Auslaßseite der Gasvolumen-Reguliervorrichtung (20;40) verbindbare Applikatorleitung (27;50).

2. Vorrichtung nach Anspruch 1, dadurch gekennzeichnet, daß die Gasvolumen-Reguliervorrichtung (20) aus einem Drosselstück (22) gebildet ist, mit dem ein zwischen Druckbehälter (10) und Drosselstück (22) eingesetztes Steuerventil (25) zusammenwirkt.

3. Vorrichtung nach Anspruch 1 oder 2, dadurch gekennzeichnet, daß das Drosselstück einen unveränderlichen Durchgangsquerschnitt aufweist.

4. Vorrichtung nach Anspruch 1 oder 2, dadurch gekennzeichnet, daß das Drosselstück einen veränderlichen Durchgangsquerschnitt aufweist.

5. Vorrichtung nach einem der Ansprüche 1 bis 4, dadurch gekennzeichnet, daß das Steuerventil (25) von Hand betätigbar ist.

6. Vorrichtung nach Anspruch 1, dadurch gekennzeichnet, daß die Gasvolumen-Reguliervorrichtung (40) aus einem Dreiwegehahn (41) mit T-förmigen Anschlüssen (43,44,45) und einem Küken (42) mit L-förmigem Durchgang (47,48) sowie einer mit einem der Anschlüsse (44) verbundenen Gas-Dosiervorrichtung (46) für Kohlendioxid gebildet ist.

7. Vorrichtung nach Anspruch 6, dadurch gekennzeichnet, daß die Gas-Dosiervorrichtung (46) eine Dosierkammer (54) mit veränderlichem Volumen aufweist.

8. Vorrichtung nach Anspruch 6 oder 7, dadurch gekennzeichnet, daß die Gas-Dosiervorrichtung als zylindrischer Hohlkörper (55) ausgebildet ist, der an einer Stirnseite einen Konnektor zur Verbindung mit dem einen Anschluß (44) des Dreiwegehahns (41) aufweist und in dem ein Kolben (56) verschiebbar ist, der in verschiedenen Positionen arretierbar ist.

9. Vorrichtung nach einem der Ansprüche 1 bis 8, dadurch gekennzeichnet, daß entweder an die Gasvolumen-Reguliervorrichtung (40) oder direkt an die Applikatorleitung (27;50) eine Gasdruck-Meßvorrichtung (60) angeschlossen ist.

10. Vorrichtung nach einem der Ansprüche 1 bis 9, dadurch gekennzeichnet, daß die Applikatorleitung eine Darmsonde (27) ist.

11. Vorrichtung nach einem der Ansprüche 1 bis 9, dadurch gekennzeichnet, daß die Applikatorleitung ein Angiographie-Katheter (50) oder eine Hohlnadel bzw. Kanüle ist.

12. Vorrichtung nach einem der Ansprüche 1 bis 11, dadurch gekennzeichnet, daß der Druckbehälter (10; 30) und die Gasvolumen-Reguliervorrichtung (20;40) als Baueinheit in einer gemeinsamen sterilen Verpackung (100) untergebracht sind.

13. Vorrichtung nach einem der Ansprüche 10 bis 12, dadurch gekennzeichnet, daß die Aplikatorleitung in die Verpackung (100) mit einbezogen ist.
